# EUROPEAN PATENT APPLICATION

(11) **EP 4 592 123 A1**
(43) Date of publication of application: **30.07.2025**
(21) Application number: 24154894.0
(22) Date of filing: 31.01.2024
(51) Int. Cl.: B60K 35/22, A61B 5/16, B60K 35/26, G10L 25/63

(54) **VEHICLE DISPLAY DEVICE FOR AI STRESS FEEDBACK**

(30) Priority: 23.01.2024 KR 20240010247
(71) Applicant: Sunmoon University Industry University Cooperation Foundation, Asan-si, Chungcheongnam-do 31460 (KR)
(72) Inventor: YUN, Jong Hwan, Seoul 04539 (KR); KANG, Min Soo, Seoul 04539 (KR)
(74) Representative: Rösler Rasch van der Heide & Partner

(57) **Abstract**

Disclosed is a vehicle display device for AI stress feedback allowing feedback according to a stress state of a driver by detecting a voice of a vehicle driver. The vehicle display device includes a voice intensity measurement value collection module that calculates an intensity value of a voice signal of a passenger, a relative location calculation module that estimates an angle between respective microphones based on an intensity difference between the microphones, a direction estimation module that estimates a direction of voice based on the angle between the microphones, a voice DNA extraction module that detects a singular point of an individual voice by analyzing a unique frequency, intensity, and tone of each voice signal, and a driver voice extraction module that collects a feature of each voice signal extracted for each utterance direction, and then derives utterance of a driver's seat to identify voice of the driver.

## Description

### BACKGROUND OF THE INVENTION

### Field of the Invention

The present invention relates to an artificial intelligence (AI) stress feedback device, and more particularly to a vehicle display device for AI stress feedback allowing driver-centered stress feedback by individually recognizing stress states of a driver and a passenger.

### Description of the Related Art

This research was supported by "Regional Innovation Strategy (RIS)" through the National Research Foundation of Korea(NRF) funded by the Ministry of Education(MOE)(2021RIS-004)
Recently, a vehicle display device includes a display, an audio output unit, a communication unit configured to exchange data with a server or an image display device, a memory configured to store location information related to content received from the server or the image display device through the communication unit, and a processor configured to reproduce content when a vehicle moves and is located at a location corresponding to location information related to the content and to perform a control operation so that the reproduced content is provided through the display or the audio output unit.

As in the attached prior literature, a vehicle 200 may be equipped with a vehicle display device 100 inside the vehicle, and exchanges data with an image display device 600 or a server 500 through an internal communication unit (not shown) of the vehicle display device 100.

In particular, data is exchanged with the image display device 600 or the server 500 through a network 570. There are a variety of examples of the image display device 600. However, in the figure, a TV 600a and a terminal 600b are illustrated. Meanwhile, the TV 600a and the terminal 600b may exchange data with the server 500 or the vehicle 200, particularly the vehicle display device 100 mounted in the vehicle. The terminal 600b may be a mobile terminal such as a mobile phone, a smartphone, a tablet computer, or a wearable device such as a smartwatch.

The server 500 is a server provided by a vehicle manufacturer or a server operated by a provider providing a vehicle-related service. For example, the server 500 may be a server operated by a provider providing information on road traffic conditions, etc. This vehicle display device 100 may store location information related to content received from the server 500 or the image display device 600 through the communication unit, reproduce the content when the vehicle moves and is located at a location corresponding to the location information related to the content, and output the reproduced content to at least one of the display or the audio output unit. Accordingly, the driver may enjoy related content without any additional manipulation.

The vehicle display device 100 may determine whether the vehicle is located at the location corresponding to the location information related to the content based on the location information on the vehicle. The vehicle display device 100 may perform a control operation so that the location corresponding to the location information related to the content is matched on a stored map for driving the vehicle, and determine whether the location information on the vehicle is located at the location corresponding to the location information related to the content matched on the map.

The vehicle display device 100 may perform a control operation so that a map screen is displayed through a display 180 while the vehicle is driving, reproduce the content when the vehicle moves and is located at the location corresponding to the location information related to the content, and output the reproduced content to at least one of the display or the audio output unit. The vehicle display device 100 may transmit user account information to the server 500 or the image display device 600, and receive content-related location information from the server 500 or the image display device 600 in response to the user account information.

The vehicle display device 100 may receive location information related to content and content data from the server 500. Accordingly, when the vehicle is located at a specific location, the content data may be reproduced and output. The location information related to the content may be location information related to content collected from the image display device 600 or the server 500 according to storage input for the content reproduced through the image display device 600.

That is, when there is a content storage command while watching the TV 600a, the TV 600a may store broadcast video being reproduced. In particular, video of specific scenes may be stored. Further, the stored scene video may be transmitted to the server 500. The server 500 may analyze the scene video, use metadata added to the scene video, or use EPG and related broadcast program-related information to extract location information from the scene video.

Further, the server 500 may directly transmit the extracted location information to the vehicle display device 100. Alternatively, the server 500 may transmit the extracted location information to the TV 600a, and the TV 600a may transmit the extracted location information to the vehicle display device 100. The terminal 600b may store radio sound being reproduced. Further, the stored radio sound may be transmitted to the server 500. The server 500 may analyze the radio sound, use metadata added to the radio sound, or use broadcast program-related information to extract location information from the radio sound.

The conventional vehicle display device configured in this way stores content based on a voice command of a user and allows content to be reproduced through the vehicle display device inside the vehicle based on location information related to the stored content, so that an effect of increasing convenience of the user is provided. However, this method of providing content corresponding to the location of the vehicle has a problem of increasing driver stress by indiscriminately providing content. Therefore, there is a need for a method to improve usefulness of content by providing appropriate content in response to a driver stress situation.

[Patent Literature] Republic of Korea Patent Publication No. 10-2016-0082060, publication date July 8, 2016, title of the invention "Display apparatus for vehicle and vehicle including the same"

### SUMMARY OF THE INVENTION

Therefore, the present invention has been made in view of the above problems, and it is an object of the present invention to provide a vehicle display device for AI stress feedback capable of promoting safe driving by extracting voice corresponding to a location of a driver from a conversation between the driver and a passenger and determining a stress state based on the voice of the driver.

In accordance with the present invention, the above and other objects can be accomplished by the provision of a vehicle display device for artificial intelligence (AI) stress feedback allowing feedback according to a stress state of a driver in conjunction with a navigation system of a vehicle, the vehicle display device including a display module that constitutes a screen of the vehicle display device and has at least two microphones installed at both ends or diagonally on the screen, a navigation server configured to provide navigation information of the vehicle to the screen of the display module in real time, an AI server configured to provide content according to user emotion corresponding to a voice pattern, a navigation controller configured to perform a navigation function necessary for vehicle operation based on communication with the navigation server, an AI controller configured to analyze voice input from the microphone to determine emotion corresponding to the driver voice, and to provide driver emotion information to the AI server to receive content according to needs and emotion of the driver, and an operating device including a communication unit wirelessly connected to the navigation server and the AI server to perform communication with each server according to navigation operation and AI operation.

The AI controller may include a driver voice recognition unit configured to extract voice originating from a driver's seat from conversation voices of a plurality of passengers based on the at least two microphones, an AI voice analyzer configured to determine a stress state of the driver extracted from the driver voice recognition unit based on AI analysis through connection to the AI server, and an input/output controller configured to provide content corresponding to the stress state of the driver derived from the AI voice analyzer to the display module or a speaker when content is provided according to a driver request.

The driver voice recognition unit may determine directionality by measuring intensity or amplitude of audio, measure audio intensity using the at least two microphones, and estimate a direction of sound therefrom.

The driver voice recognition unit may include a voice intensity measurement value collection module configured to calculate intensity values for a plurality of voice signals through the at least two microphones, a relative location calculation module configured to estimate an angle between the respective microphones based on an intensity difference between the respective microphones measured from the voice intensity measurement value collection module, a direction estimation module configured to estimate a direction of voice based on the angle between the respective microphones, a voice DNA extraction module configured to detect a singular point of an individual voice by analyzing a unique frequency, intensity, and tone of each of a plurality of voice signals through the at least two microphones, and a driver voice extraction module configured to define a direction of voice utterance from the direction estimation module, collect a feature of each voice signal extracted for each utterance direction from the voice DNA extraction module, and then derive utterance of the driver's seat to identify voice of the driver.

The voice DNA extraction module may extract and quantify each feature such as a frequency, intensity, or tone of a voice signal, and any one algorithm among Mel-Frequency Cepstral Coefficients (MFCC), Linear Predictive Coding (LPC), and Perceptual Linear Prediction (PLP) may be applied.

The AI voice analyzer may use an SVM (Support Vector Machine) algorithm to predict the stress state based on a voice feature vector of the driver extracted by the voice DNA extraction module.

### BRIEF DESCRIPTION OF THE DRAWINGS

The above and other objects, features and other advantages of the present invention will be more clearly understood from the following detailed description taken in conjunction with the accompanying drawings, in which:

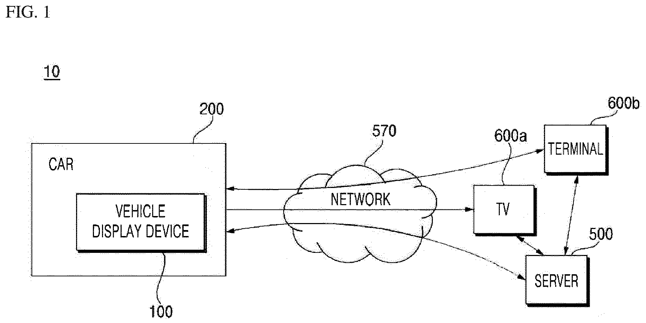

FIG. 1 is a configuration diagram for describing a display device of a conventional vehicle;
FIG. 2 is a configuration diagram for describing a vehicle display device for AI stress feedback according to the present invention; and
FIG. 3 is a configuration diagram for describing a driver voice recognition unit applied in the present invention.

### DETAILED DESCRIPTION OF THE INVENTION

Hereinafter, a preferred embodiment of the present invention will be described in detail based on the attached illustration drawings.

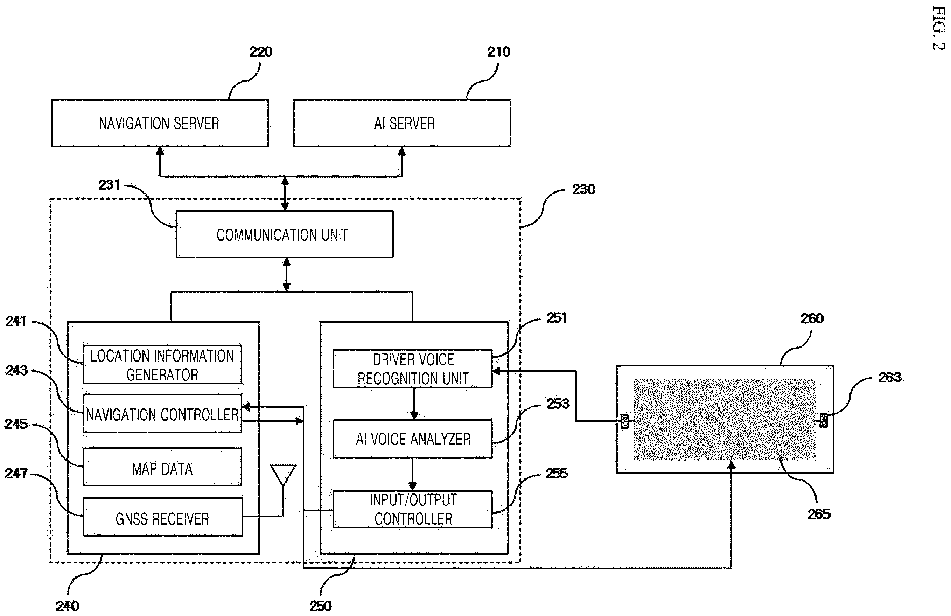

FIG. 2 is a configuration diagram for describing a vehicle display device for AI stress feedback according to the present invention. The vehicle display device presented in the present invention is illustrated as being linked to a vehicle navigation system. However, the vehicle display device may be implemented as a separate device as needed. Therefore, the vehicle display device according to the present invention implements a navigation function of a typical vehicle.

As shown in the figure, the vehicle display device includes a display module 260 that constitutes a screen 265 of the vehicle display device and has at least two microphones 263 installed at both ends or diagonally on the screen 265, a navigation server 220 that provides navigation information of the vehicle to the screen 265 of the display module 260 in real time, an AI server 210 that provides content according to user emotion corresponding to a voice pattern, and an operating device 230 having a navigation controller 240 that performs a navigation function necessary for vehicle operation based on communication with the navigation server 220 and an AI controller 250 that analyzes voice input from the microphone 263 to determine emotion corresponding to the driver voice and provides driver emotion information to the AI server 210 to receive content according to needs and emotion of the driver.

The operating device 230 includes a communication unit 231 for performing communication with each of the navigation server 220 and the AI server 210, and the communication unit 231 performs communication with each server according to navigation operation and AI operation. That is, during navigation operation, the communication unit 231 is connected to the navigation server 220 through a wide area network such as the Internet to receive information on a guidance route from the navigation server 220, and provides route guidance for the vehicle based on the received information.

Meanwhile, when the communication unit 231 receives AI content, the communication unit 231 communicates with the AI server 210 and receives content corresponding to the driver emotion. Here, the driver is a subject who directly operates the vehicle, stores voice of the driver not to determine voice of the driver, and refers to a subject who drives the vehicle. In other words, the driver refers to a user located in a driver's seat, and in the present invention, voice of the driver among a plurality of passengers is recognized and emotion of the driver are determined to provide content corresponding to the emotion.

The navigation controller 240 includes a location information generator 241 that extracts location information of the vehicle in real time, a navigation controller 243 that provides geographic guidance to a set location based on map data 245 registered in a memory thereof, and a GNSS receiver 247 that receives location information of the vehicle.

The GNSS receiver 247 receives radio waves from a satellite, provides information extracted from the radio waves to the navigation controller 243, outputs the map data 245 indicating a route to a destination based on the provided information to the display module 260, and outputs voice for route guidance to a speaker.

The location information generator 241 obtains a location of the vehicle (for example, coordinates on the Earth) based on information from various sensors or the GNSS receiver 247 and captures the location of the vehicle on a road. The location information generator 241 combines the map data 245 and the location of the vehicle to capture a link ID of the road on which the vehicle is traveling. The location information generator 241 matches vehicle location information indicating the captured vehicle location with date and time information indicating a date and time at which the vehicle location is captured, and transmits the information to the navigation server 220 via the communication unit 231.

The location information generator 241 acquires a speed of the vehicle and transmits speed information indicating the speed of the vehicle to the navigation server 220 via the communication unit 231. Therefore, the vehicle speed is calculated based on a travel distance indicated by the vehicle location information and a travel time.

The navigation controller 243 generates a map image representing a driving location of the vehicle based on the map data 245 stored in advance and the vehicle location information generated by the location information generator 241, and searches for a route to the destination based on the vehicle location information. The navigation controller 243 generates a map image that displays the route obtained through the search by overlaying the route on the map, and sets a navigation route according to a user request.

When the route obtained through the search is set by the user as a route for navigating the vehicle, the navigation controller 243 transmits destination information along with the vehicle location information to the navigation server 220.

Meanwhile, the AI controller 250 includes a driver voice recognition unit 251 that extracts voice originating from the driver's seat from conversation voices of a plurality of passengers based on the at least two microphones 263, an AI voice analyzer 253 that determines a stress state of the driver extracted from the driver voice recognition unit 251 based on AI analysis through connection to the AI server 210, and an input/output controller 255 that provides content corresponding to the stress state of the driver derived from the AI voice analyzer 243 to the display module 260 or the speaker when content is provided according to a driver request.

The driver voice recognition unit 251 derives a voice signal of the driver by analyzing an audio signal input from the at least two microphones 263, and extracts the voice of the driver based on each seat location of the passenger.

The driver voice recognition unit 251 measures a time difference between sound arrivals using the at least two microphones 263, and it is possible to apply Time Difference of Arrival (TDOA), in which a distance from a point where voice originates to each microphone 263 is calculated and a direction of sound is determined based thereon.

In addition, the driver voice recognition unit 251 determines directionality using voice frequency changes, and it is possible to apply Frequency Difference of Arrival (FDOA), in which voice frequency changes input from the at least two microphones 263 are measured and a direction of sound is measured based thereon, thereby extracting voice of the driver.

Meanwhile, in the present invention, the driver voice recognition unit 251 determines directionality by measuring intensity or amplitude of audio, measures audio intensity using the at least two microphones 263, and estimates a direction of sound therefrom.

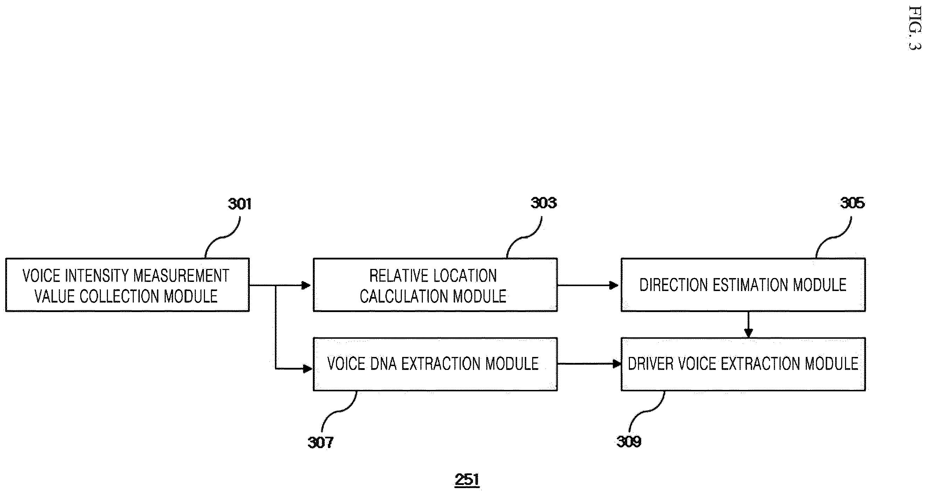

FIG. 3 is a configuration diagram for describing main functions of the driver voice recognition unit 251 applied in the present invention.

First, in the present invention, the at least two microphones 263 are installed on an outer peripheral surface of the display module 250, which means that four microphones 263 may be installed on a vertical line or two microphones 263 may be arranged on a horizontal line.

As shown in the figure, the driver voice recognition unit 251 includes a voice intensity measurement value collection module 301 that calculates intensity values for a plurality of voice signals through the at least two microphones 263, a relative location calculation module 303 that estimates an angle between the respective microphones 263 based on an intensity difference between the respective microphones 263 measured from the voice intensity measurement value collection module 301, a direction estimation module 305 that estimates a direction of voice based on the angle between the respective microphones 263, a voice DNA extraction module 307 for detecting a singular point of an individual voice by analyzing a unique frequency, intensity, and tone of each of a plurality of voice signals through the at least two microphones 263, and a driver voice extraction module 309 that defines a direction of voice utterance from the direction estimation module 305, collects a feature of each voice signal extracted for each utterance direction from the voice DNA extraction module 307, and then derives utterance of the driver's seat to identify voice of the driver.

In measuring voice intensity by calculating amplitude of the audio signal measured by the at least two microphones 263, the voice intensity measurement value collection module 301 may calculate voice intensity by squaring the amplitude of the voice signal, or calculate voice intensity by applying log transformation to the amplitude of the voice signal.

The voice DNA extraction module 307 may use any one algorithm among Mel-Frequency Cepstral Coefficients (MFCC), Linear Predictive Coding (LPC), and Perceptual Linear Prediction (PLP) when extracting and quantifying each feature such as a frequency, intensity, or tone of the voice signal.

Hereinafter, an operation of the vehicle display device for AI stress feedback according to the present invention will be described. First, an operation of the navigation controller 240 is similar to that of a typical vehicle navigation system, and thus a detailed operation description will be omitted. Meanwhile, an operation of the AI controller 250 is described as follows.

The at least two microphones 263 are installed horizontally or vertically on the sides of the screen 265 of the display module 260, and a voice signal of the passenger flowing into each microphone 263 is provided to the driver voice recognition unit 251.

The driver voice recognition unit 251 detects voice directionality of passengers, including the driver, in the vehicle. As described above, it is possible to apply Time Difference of Arrival (TDOA), in which a distance from a point where voice originates to each microphone 263 is calculated and a direction of sound is determined based thereon, or it is possible to apply Frequency Difference of Arrival (FDOA), in which a change in frequency of sound is measured and a direction of the sound is estimated based thereon.

In the present invention, a method of measuring each voice intensity or amplitude and identifying directionality is applied, in which intensity of voice is measured using a plurality of microphones 263 and a direction of sound is estimated based thereon. That is, a voice signal from the surrounding environment of each microphone 263 is collected through the voice intensity measurement value collection module 301.

With regard to the voice signal collected in this way, intensity of each signal is calculated. The voice intensity may be calculated by squaring the amplitude of the voice signal, or the voice intensity may be calculated by applying log transformation to the amplitude of the audio signal. Thereafter, the relative location calculation module 303 estimates the angle between the microphones 263 by calculating relative locations between the microphones 263 based on the calculated voice intensity.

Thereafter, the direction estimation module 305 estimates a direction of voice based on the angle between the respective microphones 263 and expresses the angle using a circular coordinate system. In other words, an estimated location of voice occurrence is converted into a direction through an algorithm, and a direction of sound is calculated within a 360-degree range. As the at least two microphones 263 applied in the present invention are installed on a straight line, the location of the voice occurrence is measured at 180 degrees, making it easy to identify the location of the voice.

Meanwhile, the voice DNA extraction module 307 extracts a voice feature for each of a plurality of voice signals collected by the voice intensity measurement value collection module 301. To this end, the voice DNA extraction module 307 converts the collected voice signal into digital form and performs processing such as noise removal and filtering. Then, a voice feature is extracted from each voice signal. As described above, each feature such as a frequency, intensity, or tone of the voice signal is extracted and quantified based on any one algorithm among MFCC, LPC, and PLP.

Thereafter, the voice DNA extraction module 307 reduces the amount of calculation by reducing dimensions of a feature vector using technique such as principal component analysis (PCA), and derives important information, thereby classifying each of voices of the passengers based on the voice feature vector.

The driver voice extraction module 309 identifies the voice originating from the driver's seat based on voice classification information for each passenger derived from the voice DNA extraction module 307 and direction information for each passenger voice extracted from the direction estimation module 305, and selects a voice feature vector for the corresponding voice.

Therefore, the voice uttered from the driver's seat is recognized, and only the voice of the driver is extracted from a conversation between passengers in the vehicle based on a feature of the voice. In this way, the driver voice recognition unit 251 determines the voice of the driver during the conversation between passengers, and recognizes a voice of a current driver even when the driver is replaced. In other words, in the present invention, rather than recognizing a voice of a specific person among the passengers, the voice of the current driver is recognized, and a stress situation of the driver is determined, so that driver-centered content is provided.

When the voice of the driver is recognized by the driver voice recognition unit 251, a stress state of the driver is determined through the AI voice analyzer 253. The AI voice analyzer 253 uses an SVM algorithm to predict the stress state based on the voice feature vector of the driver extracted by the voice DNA extraction module 307, which means predicting a stress state of new voice data using a trained model, and voice therapy, stress management, and emotion analysis are performed.

The SVM algorithm is a machine learning algorithm and is a method of finding an optimal decision boundary by mapping data into a high-dimensional space. In other words, the SVM algorithm is an algorithm that finds a decision boundary to classify data, and is trained to maximize a margin between some data points referred to as support vectors and the decision boundary.

In other words, the SVM algorithm finds an optimal decision boundary in data mapped to a high-dimensional space, this decision boundary is determined using a method of maximizing a margin with a support vector, and a new data value is predicted by performing training so that data points are located around the decision boundary. Here, for emotional analysis of the voice of the driver, the SVM algorithm performs mapping to a high-dimensional space using a kernel technique and is used as a function to calculate a similarity between data points.

The kernel technique may be a polynomial kernel or a Gaussian kernel. The polynomial kernel is used to consider nonlinear features of text data, and maps input data to a high-dimensional space to find a nonlinear decision boundary. In addition, the polynomial kernel finds a nonlinear decision boundary by mapping using a polynomial function of a given degree, which extracts changes in the voice of the driver and recognizes a previously learned stress state based on the changes in the voice.

Meanwhile, the Gaussian Kernel is applied as a kernel for processing a nonlinear problem, and calculates a similarity between data points using a Gaussian distribution. An embedding vector of a word is used in consideration of features of text data, and a similarity between vectors is calculated using the Gaussian kernel.

In this way, as the AI voice analyzer 253 analyzes the stress state of the driver corresponding to the voice of the driver and provides the stress state to the AI server 210, the AI server 210 provides content according to the stress state of the driver. It is obvious that content is created and provided according to a request from the driver, and the AI server 210 provides the content to the speaker or the display module 260 of the vehicle through the input/output controller 255.

Providing content according to a stress state means providing appropriate content according to a stress level of the driver. For example, when the driver feels stressed, content that provides a stable and calm atmosphere may be selected, and content such as meditation, quiet music, or natural scenery photos may be provided to calm the mind of the driver.

In addition, when psychological support is needed to manage stress of the driver, it is possible to recommend a stress management method, a healing-related article, video, a self-development book, etc. to the driver, and to provide a wise saying for stress management or content that conveys positive energy. Depending on the need, a humorous video, a funny story, information about a hobby, etc. may be provided to the driver when laughter and fun are needed to relieve stress.

The vehicle display device for AI stress feedback presented by the present invention provides an effect of being able to promote safe driving by extracting voice corresponding to a location of a driver from a conversation between the driver and a passenger and determining a stress state based on the voice of the driver.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A vehicle display device for artificial intelligence (AI) stress feedback allowing feedback according to a stress state of a driver in conjunction with a navigation system of a vehicle, the vehicle display device comprising:
a display module (260) that constitutes a screen (265) of the vehicle display device and has at least two microphones (263) installed at both ends or diagonally on the screen (265);
a navigation server (220) configured to provide navigation information of the vehicle to the screen (265) of the display module (260) in real time;
an AI server (210) configured to provide content according to user emotion corresponding to a voice pattern;
a navigation controller (240) configured to perform a navigation function necessary for vehicle operation based on communication with the navigation server (220);
an AI controller (250) configured to analyze voice input from the microphone (263) to determine emotion corresponding to the driver voice, and to provide driver emotion information to the AI server (210) to receive content according to needs and emotion of the driver; and
an operating device (230) including a communication unit (231) wirelessly connected to the navigation server (220) and the AI server (210) to perform communication with each server according to navigation operation and AI operation.

2. The vehicle display device according to claim 1, wherein the AI controller (250) comprises:
a driver voice recognition unit (251) configured to extract voice originating from a driver's seat from conversation voices of a plurality of passengers based on the at least two microphones (263);
an AI voice analyzer (253) configured to determine a stress state of the driver extracted from the driver voice recognition unit (251) based on AI analysis through connection to the AI server (210); and
an input/output controller (255) configured to provide content corresponding to the stress state of the driver derived from the AI voice analyzer (243) to the display module (260) or a speaker when content is provided according to a driver request.

3. The vehicle display device according to claim 2, wherein the driver voice recognition unit (251) determines directionality by measuring intensity or amplitude of audio, measures audio intensity using the at least two microphones (263), and estimates a direction of sound therefrom.

4. The vehicle display device according to claim 3, wherein the driver voice recognition unit (251) comprises:
a voice intensity measurement value collection module (301) configured to calculate intensity values for a plurality of voice signals through the at least two microphones (263);
a relative location calculation module (303) configured to estimate an angle between the respective microphones (263) based on an intensity difference between the respective microphones (263) measured from the voice intensity measurement value collection module (301);
a direction estimation module (305) configured to estimate a direction of voice based on the angle between the respective microphones (263);
a voice DNA extraction module (307) configured to detect a singular point of an individual voice by analyzing a unique frequency, intensity, and tone of each of a plurality of voice signals through the at least two microphones (263); and
a driver voice extraction module (309) configured to define a direction of voice utterance from the direction estimation module (305), collect a feature of each voice signal extracted for each utterance direction from the voice DNA extraction module (307), and then derive utterance of the driver's seat to identify voice of the driver.

5. The vehicle display device according to claim 4, wherein the voice DNA extraction module (307) extracts and quantifies each feature such as a frequency, intensity, or tone of a voice signal, and any one algorithm among Mel-Frequency Cepstral Coefficients (MFCC), Linear Predictive Coding (LPC), and Perceptual Linear Prediction (PLP) is applied.

6. The vehicle display device according to claim 4, wherein the AI voice analyzer (253) uses an SVM (Support Vector Machine) algorithm to predict the stress state based on a voice feature vector of the driver extracted by the voice DNA extraction module (307).
